# EUROPÄISCHE PATENTSCHRIFT

(11) **EP 2 446 290 B1**
(45) Veröffentlichungstag und Bekanntmachung des Hinweises auf die Patenterteilung: **19.12.2018**
(21) Anmeldenummer: 10732328.9
(22) Anmeldetag: 23.06.2010
(51) Int. Cl.: G01R 33/383, G01R 33/3875

(54) **MAGNETFELDEINHEIT EINES MRT-SYSTEMS ZUR BILDGEBENDEN ERFASSUNG EINES KOPFBEREICHS**
MAGNETIC FIELD UNIT OF AN MRI SYSTEM FOR IMAGE CAPTURING A HEAD REGION
UNITÉ DE CHAMP MAGNÉTIQUE D'UN SYSTÈME IRM PERMETTANT L'ACQUISITION D'UNE IMAGE D'UNE RÉGION DE LA TÊTE

(30) Priorität: 23.06.2009 DE 102009027119
(43) Veröffentlichungstag der Anmeldung: 02.05.2012
(73) Patentinhaber: Sirona Dental Systems GmbH, 64625 Bensheim (DE)
(72) Erfinder: RASCHE, Volker, 89155 Erbach (DE); HELL, Erich, 66557 Illingen (DE); ULRICI, Johannes, 64293 Darmstadt (DE)
(74) Vertreter: Sommer, Peter
(86) Internationale Anmeldenummer: PCT/EP2010/058875
(87) Internationale Veröffentlichungsnummer: WO 2010/149686

(56) Entgegenhaltungen:
- EP-A1- 0 067 933
- EP-A1- 0 654 675
- DE-A1- 4 239 048
- DE-A1-102007 038 382
- GB-A- 2 319 339
- JP-A- 63 286 142
- JP-A- 2003 334 175
- US-B1- 7 102 353
- SWARTZ ET AL: "In vivo EPR for dosimetry" RADIATION MEASUREMENTS, ELSEVIER, AMSTERDAM, NL LNKD- DOI:10.1016/J.RADMEAS.2007.05.023, Bd. 42, Nr. 6-7, 1. Juli 2007 (2007-07-01) , Seiten 1075-1084, XP022264801 ISSN: 1350-4487
- DATABASE WPI Week 199925 Thomson Scientific, London, GB; AN 1999-295447 XP002604022 -& JP 11 099134 A (NONOMURA Y) 13. April 1999 (1999-04-13)

## Beschreibung

### Technisches Gebiet

Die Erfindung betrifft eine Magnetfeldeinheit eines MRT-Systems zur bildgebenden Erfassung eines Teils eine Kopfes als aufzunehmender Bereich, wobei die Magnetfeldeinheit eine Permanentmagnetanordnung, eine Vorrichtung zur Erzeugung eines Gradientenfeldes zur Erzeugung eines Gradientenfeldes und mindestens eine Spule zum Erzeugen und Empfangen von Hochfrequenzen umfasst.

### Stand der Technik

Im Bereich der Zahnmedizin wird die Magnetresonanztomographie bisher nur sehr eingeschränkt genutzt. Dies liegt vor allem an den hohen Kosten von Installation und Betrieb sowie an den limitierten dentalen klinisch relevanten Bildinformationen.

Weit verbreitet sind dahingegen Röntgenaufnahmen, wie sie beispielsweise mit der aus der EP 0 632 994 A1 bekannten Vorrichtung vorgenommen werden können. Dort wird eine Röntgendiagnotikeinrichtung zur Erstellung von Röntgenaufnahmen eines Körperteils, beispielsweise eines Kiefers, offenbart, wobei eine Zeilendetektor-Kamera und eine diametral gegenüber angeordnete Strahlenquelle synchron um das aufzunehmende Objekt bewegbar sind.

Die Verwendung der Magnetresonanztomographie zur Bilderzeugung bietet gegenüber der klassischen Röntgenaufnahme den Vorteil, dass der Patient keiner Strahlenbelastung mit Röntgenstrahlen ausgesetzt werden muss.

Aus der EP 1876 462 A1 ist beispielsweise Magnetsystem für Elektronenspinresonanz-Untersuchungen von Proben bekannt, welches um einem Probenraum herum angeordnet ist und innerhalb des Probenraums ein Magnetfeld mit variabler Feldstärke erzeugt.

Aus dem Artikel "In Vivo MRI-Based Dental Impression Using an Intraoral Rf Receiver Coil" von P. M. Jakob, erschienen in Concepts in Magnetic Resonance Part B, Magnetic Resonance Engineering, Vol. 33 B(4) 244-251, 2008, ist eine Empfangsspule für Hochfrequenzen eines MRT-Systems bekannt, die in den Mund des Patienten einbringbar ist und innerhalb eines herkömmlichen MRT-Apparts verwendet werden kann. Dadurch kann die Empfangsspule möglichst nah an dem Aufzunehmenden Objekt, nämlich dem Kiefer und/oder den Zähnen positioniert werden, wobei die Qualität einer Aufnahme des Kieferbereichs mit einem herkömmlichen MRT-Apparat verbessert werden kann.

Aus der DE 10 2007 038 382 ist ein Oberkörper-Magnetresonanztomographiegerät mit einer offenen Magnetanordnung, z.B. einem C-förmigen Permanentmagneten, zur Erzeugung eines Hauptmagnetfelds bekannt, welches zur Patientenpositionierung eine Griffstangen, ein aufblasbares Kissen und eine Patientenplattform mit einer Hubeinheit aufweist, wobei der Patient mittels der Hubeinheit in vertikaler Richtung relativ zum Magneten bewegbar ist. Anspruch ist gegenüber diesem Dokument abgegrenzt worden.

Aus der US 7,102,353 B1 ist ein Hauptmagnet eines MRT-Systems bekannt, welches sich zur Vermessung des Kopfbereichs eines Patienten eignet, wobei der Hauptmagnet entweder als offener Permanentmagnet oder als geschlossener Elektromagnet ausgebildet und relativ zum Patienten bewegbar ist.

Gemäß dem Artikel "In vivo EPR for dosimetry" von H. M. Swartz et al. wird eine offene Permanentmagnetanordnung für eine Elektronenspinresonanzuntersuchung verwendet.

Aus der JP 63-286142 ist eine offene Magnetanordnung aus zwei an einem U-förmigen Joch gehaltenen Permanentmagneten bekannt. Diese Magnetanordnung wird über ein Gelenk an einem Stativ gehalten, so dass die Magnetanordnung gegenüber dem Stativ gedreht bzw. verkippt werden kann.

Aufgabe der Erfindung ist es eine in seiner Systemarchitektur angepasste, kostengünstige Magnetfeldeinheit eines MRT-Systems bereitzustellen, die eine sinnvolle Verwendung der Magnetresonanztomographie insbesondere im Bereich der Zahnmedizin ermöglicht.

### Darstellung der Erfindung

Die erfindungsgemäße Magnetfeldeinheit eines MRT-Systems zur bildgebenden Erfassung eines Kopfbereichs als aufzunehmender Bereich umfasst eine Permanentmagnetanordnung zur Erzeugung eines Hauptmagnetfeldes, eine Halterung für die Permanentmagnetanordnung, eine aus mehreren Spulen bestehende Vorrichtung zur Erzeugung eines Gradientenfeldes, sowie mindestens eine Spule zum Erzeugen und Empfangen von Hochfrequenzen, wobei sich innerhalb des von der Permanentmagnetanordnung eingeschlossenen Volumens ein dreidimensionaler homogener Bereich des Hauptmagnetfeldes ausbildet und wobei die Permanentmagnetanordnung so an der Halterung befestigt ist, dass eine Längsachse der Permanentmagnetanordnung mit einer in vertikaler Richtung verlaufenden Achse einem Winkel von maximal ±45° einschliesst, so dass erreicht werden kann, dass die Längsachse der Permanentmagnetanordnung und eine Hochachse eines sitzenden oder stehenden Patienten maximal einen Winkel von 40° einschliessen, wobei die Permanentmagnetanordnung und der Patient relativ zueinander so verstellbar sind, dass die Magnetfeldeinheit in eine Lage um den Kopf des Patienten herum gebracht werden kann. Die Permanentmagnetanordnung setzt sich aus axial beabstandeten Permanentmagneten mit jeweils einer im Querschnitt senkrecht zu der Längsachse geschlossenen Form zusammen, und die Spulen der Vorrichtung zur Erzeugung des Gradientenfelds und die mindestens eine Spule zum Erzeugen und Empfangen von Hochfrequenzen sind in Zwischenräumen zwischen den axial beabstandeten Permanentmagneten angeordnet.

Der Vorteil einer vertikalen Ausrichtung der erfindungsgemäßen Magnetfeldeinheit eines MRT-Systems ist, dass der Patient im Sitzen oder Stehen vermessen werden kann und sich nicht hinlegen muss. Es besteht dadurch sogar die Möglichkeit, den Patienten während des Vermessens auf einem Zahnarztstuhl zu positionieren, der in jeder Zahnarztpraxis vorhanden ist.

Vorteilhafterweise weist ein von der Permanentmagnetanordnung zumindest teilweise begrenztes Volumen einen Durchmesser auf, der maximal dem zweifachen eines Durchmessers eines Normkopfes entspricht. Dadurch wird sichergestellt, dass der Kopf eines Patienten gut innerhalb der Permanentmagnetanordnung positionierbar ist, diese aber so kompakt wie möglich gebaut wird. Durch die Kompaktheit wird möglichst wenig magnetisches Material benötigt, was die Kosten gering hält und ein möglichst geringes Gewicht erreicht, was eine besonders leichte Handhabbarkeit erlaubt.

Vorteilhafterweise ist der homogene Bereich des erzeugten Hauptmagnetfeldes mindestens so groß, dass er die unteren 15 cm eines in der Permanentmagnetanordnung positionierten Normkopfes abdeckt. So wird sichergestellt, dass eine MRT-Aufnahme des Kieferbereichs eines Patienten vorgenommen werden kann.

Es kann beispielsweise eine Halbachgeometrie für die Permanentmagneten gewählt werden. Dies ermöglichte es mit einem geringen Bedarf an Magnetmaterial und mit einem entsprechend geringen Gewicht der Vorrichtung ein Hauptmagnetfeld zu erzeugen. Die geschlossene Geometrie sorgt weiterhin dafür, dass das Streufeld des Hauptmagnetfeldes klein ist. Vorteilhafterweise ist die geschlossene Form länger als breit. Dadurch wird ein homogener Bereich erzeugt, der ebenfalls eine längliche Form aufweist, wodurch dieser mit dem Kieferbereich des Patienten in Überlappung gebracht werden kann und die gesamten Ausmasse des Permanentmagnete und damit auch die Menge des verwendeten magnetischen Materials möglichst gering gehalten werden kann. Dadurch können nicht nur Kosten und Platz eingespart werden, auch das Bewegen der Magnetfeldeinheit wird durch ein geringeres Gewicht vereinfacht.

Vorteilhafterweise wird die geschlossene Form so um den Patienten herum angeordnet, dass die Hochachse des Patienten, also die Achse durch den Flächenschwerpunkt der Frankfurter Horizontalen, zum Flächenschwerpunkt einer zur Längsachse senkrechten Schnittfläche des homogenen Bereichs des Hauptmagnetfeldes beabstandet ist.

Da der zu vermessende Bereich, nämlich der Kieferbereich eines Patienten, zur Hochachse des Patienten beabstandet ist, erreicht man so eine möglichst große Überlappung des homogenen Bereichs des Magnetfeldes mit dem Kieferbereich. Vorteilhafterweise stimmt der Flächenschwerpunkt einer zur Längsachse senkrechten Schnittfläche des homogene Bereichs des Hauptmagnetfeldes mit dem Flächenschwerpunkt einer zur Längsachse senkrechten Schnittfläche des von der Permanentmagnetanordnung eingeschlossenen Volumens überein.

Dadurch kann eine recht einfache geometrische Form für die Permanentmagneten gewählt werden, beispielsweise ein Ellipsoid mit einer Wanddicke, die punktsymmetrisch zum Flächenschwerpunkt der eingeschlossenen Fläche ist. So wird auch weniger magnetisches Material benötigt, wodurch die Vorrichtung kostengünstiger und mit geringerem Gewicht hergestellt werden kann.

Vorteilhafterweise wird die geschlossene Form so um den Patienten herum angeordnet, dass die Hochachse des Patienten zum Flächenschwerpunkt einer zur Längsachse senkrechten Schnittfläche des von den Permanentmagneten eingeschlossenen Volumens beabstandet ist.

Dadurch kann für einen um den Flächenschwerpunkt einer Schnittfläche des eingeschlossenen Volumens herum angeordneten homogenen Bereich des Magnetfeldes eine möglichst große Überlappung mit dem zu vermessenden Kieferbereich des Patienten erreicht werden.

Vorteilhafterweise ist der Flächenschwerpunkt einer zur Längsachse senkrechten Schnittfläche des homogenen Bereichs des Hauptmagnetfeldes zum Flächenschwerpunkt einer zur Längsachse senkrechten Schnittfläche des von den Permanentmagneten eingeschlossenen Volumens beabstandet.

Dies ermöglicht es, den Patienten mittig innerhalb der geschlossenen Form des Permanentmagneten zu positionieren und trotzdem eine große Überlappung des homogenen Bereichs mit dem zu vermessenden Kieferbereich des Patienten zu erhalten.

Vorteilhafterweise wird die geschlossene Form so um den Patienten herum angeordnet, dass die Hochachse des Patienten mit der Lage des Flächenschwerpunktes einer zur Längsachse senkrechten Schnittfläche des von den Permanentmagneten eingeschlossenen Volumens übereinstimmt oder dieser nahe liegt, so dass der aufzunehmende Bereich, welcher zur Hochachse des Patienten beabstandet ist mit der Lage des homogenen Bereichs des Hauptmagnetfeldes übereinstimmt.

Das mittige Positionieren des Patienten ermöglicht es, die Ausmasse der Permanentmagneten möglichst gering zu halten, womit geringere Kosten, sowie ein geringeres Gewicht erreicht werden können.

Insgesamt ist es wichtig, dass der zu vermessende Bereich möglichst vollständig im homogenen Bereich des Hauptmagnetfeldes positionierbar ist. Daraus ergibt sich dann die entsprechend notwendige relative Positionierung des Patienten zum homogenen Bereich des Hauptmagnetfeldes und je nach Position des Hauptmagnetfeldes innerhalb der Magnetfeldeinheit auch die notwendige relative Position des Patienten zur Magnetfeldeinheit.

Soll der Kieferbereich eines Patienten vermessen werden, so handelt es sich um einen Bereich des Patietenkopfes, der zur Hochachse des Patienten beabstandet ist. Positioniert man den Patienten so, dass der Kieferbereich möglichst vollständig im homogenen Bereich des Hauptmagnetfeldes liegt, so ist also die Hochachse des Patienten auf alle Fälle zum Flächenschwerpunkt einer zur Längsachse der Magnetfeldeinheit senkrechten Fläche beabstandet. Je nachdem, ob der homogene Bereich des Hauptmagnetfeldes mittig oder eher am Rand der Magnetfeldeinheit positioniert ist, muss also der Patient zur Mitte der Magnetfeldeinheit verschoben, also die Hochachse des Patienten zur Längsachse der Magnetfeldeinheit beabstandet, oder genau Mittig in der Magnetfeldeinheit positioniert werden.

Vorteilhafterweise kann die Permanentmagnetanordnung mittels eines Gelenks gegenüber der Halterung um eine horizontale Achse verkippt werden.

Dadurch kann der zu vermessende Kieferbereich des Patienten besser mit dem homogenen Bereich des Magnetfeldes des Permanentmagneten zur Überlappung gebracht werden. Vorteilhafterweise sind mindestens zwei über ein Joch verbundene Permanentmagneten zur Erzeugung des Hauptmagnetfelds vorgesehen, die um den aufzunehmenden Bereich herum angeordnet sind.

Diese zu einer Seite offene Bauweise ermöglicht ein einfachs Positionieren des Patienten, der gegebenenfalls sogar während der Aufnahme ein freies Blickfeld hat. Weiterhin kann so ein größerer homogener Bereich des Magnetfelds und damit ein größerer vermessbarer Bereich erzeugt werden. Vorteilhafterweise weist die Magnetfeldeinheit eine Vorrichtung zur Fixierung eines Kopfes eines Patienten und/oder einen Aufbiss auf. Eine solche Vorrichtung kann beispielsweise eine Stirnanlage und/oder auch Ohroliven umfassen und verhindert eine Bewegung des zu vermessenden Bereichs des Patienten während einer Vermessung. Auch ein Aufbiss stellt eine Möglichkeit dar eine entsprechende Bewegung zu verhindern oder zumindest einzuschränken.

Vorteilhafterweise weist die Magnetfeldeinheit eine bewegliche Abschirmvorrichtung auf, die so um die Permanentmagnetanordnung und den an der Magnetfeldeinheit positionierten Patienten herum bringbar ist, dass möglichst keine HF-Frequenzen von aussen in den Bereich innerhalb der Abschirmvorrichtung eindringen können. Dadurch wird die Qualität der Messsignale erhöht.

Vorteilhafterweise weist die Magnetfeldeinheit ein Ausgleichsspulensystem zum Ausgleichen von Störungen des Hauptmagnetfeldes auf. Dadurch können Störungen des Hauptmagnetfeldes, die beispielsweise auch durch den Patienten hervorgerufen werden können, ausgeglichen werden. Es ist beispielsweise auch möglich Einschränkungen der Homogenität des mit der Vorrichtung erzeugten Hauptmagnetfelds beispielsweise aus Kostengründen oder baulicher Gegebenheiten in Kauf zu nehmen und mit dem Ausgleichsspulensystem zu kompensieren.

Vorteilhafterweise ist die Magnetfeldeinheit an einem Stativ in vertikaler Richtung beweglich angebracht.

Dadurch kann der Permanentmagnet um einen unterhalb der Magnetfeldeinheit sitzenden oder stehenden Patienten herum gebracht werden. Ausserdem kann die Position der Magnetfeldeinheit in vertikaler Richtung an die Größe des Patienten angepasst werden.

### Kurzbeschreibung der Zeichnung

Es zeigt die
- Fig. 1: eine Magnetfeldeinheit, die
- Fig. 2A: einen Schnitt durch die Magnetfeldeinheit aus Fig. 1, die
- Fig. 3: eine Vorrichtung zur Fixierung eines Patienten innerhalb der Magnetfeldeinheit aus Fig. 1, die
- Fig. 4: einen Permanentmagneten der Magnetfeldeinheit aus Fig. 1, die
- Fig. 5: die mit einem Gelenk versehene Magnetfeldeinheit aus Fig. 1, die

### Ausführungsbeispiele

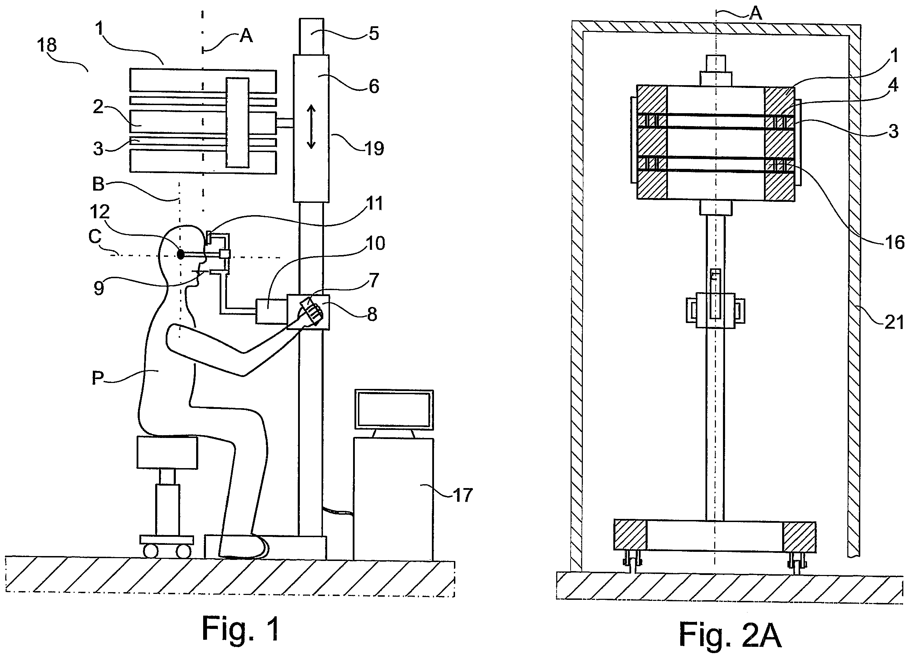

In Fig. 1 ist ein MRT-System 18 umfassend eine Magnetfeldeinheit 1 und eine Steuereinheit 17 als seitliche Ansicht abgebildet. Die Magnetfeldeinheit 1 weist mehrere Permanentmagnete 2 mit einer geschlossenen Form zur Erzeugung eines Hauptmagnetfelds sowie eine aus mehreren Spulen bestehende Vorrichtung 3 zur Erzeugung eines Gradientenfeldes und Spulen zum Erzeugen und Empfangen von Hochfrequenzen 4 auf. Eine beispielhafte Anordnung der Vorrichtung zur Erzeugung eines Gradientenfeldes 3 und der Spule zum Erzeugen und Empfangen von Hochfrequenzen 4 ist in Fig. 2A zu sehen, die einen Schnitt der erfindungsgemäßen Magnetfeldeinheit 1 darstellt. Die Spulen der Vorrichtung 3 zur Erzeugung eines Gradientenfeldes und die mindestens eine Spule 4 zum Erzeugen und Empfangen von Hochfrequenzen sind dabei in Zwischenräumen zwischen den mehreren Permanentmagneten 2 angeordnet.

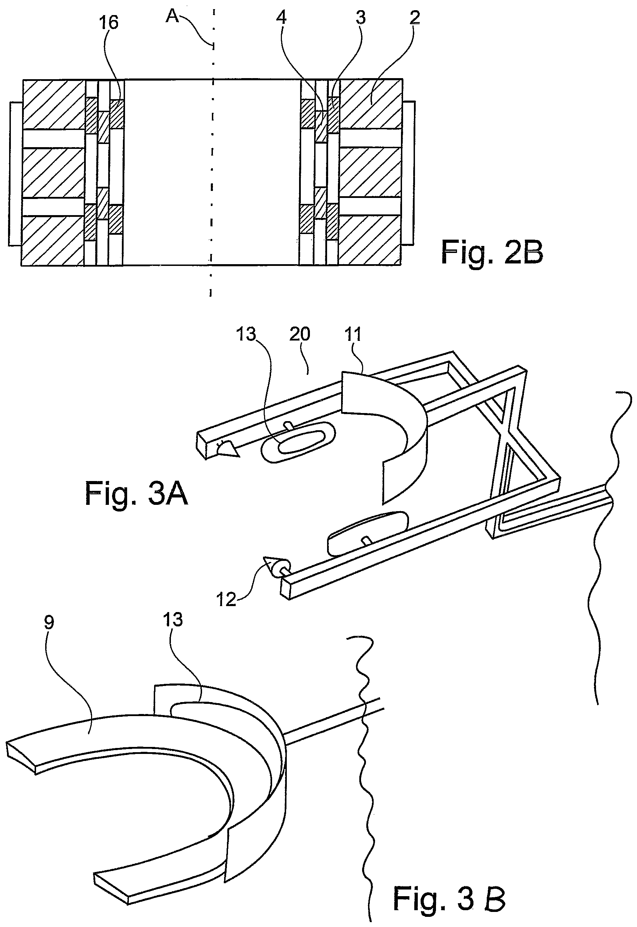

Zum Ausgleichen von Störungen des Hauptmagnetfeldes kann ausserdem noch ein Ausgleichsspulensystem 16 vorgesehen werden, welches beispielsweise aus einer ebenfalls auf einem Zylinder innerhalb des Permanentmagneten angeordneten Spule bestehen kann, wie in Fig. 2B gezeigt oder wie in Fig. 2A dargestellt in den Zwischenräumen zwischen den mehreren Permanentmagneten 2 angeordnet werden kann.

Um Störungen des Systems durch die Umgebung zu verhindern, kann eine Abschirmvorrichtung 21, wie in Fig. 2A dargestellt, vorgesehen werden, die sowohl die Magnetfeldeinheit 1 als auch den positionierten Patienten vollständig in einem Innenraum einschliesst und diesen Innenraum gegen elektromagnetische Strahlung aus der Umgebung abschirmt.

Die Magnetfeldeinheit 1 ist an einer als Stativ 5 ausgebildeten Halterung 19 in vertikaler Richtung über eine Führung 6 verschiebbar angebracht. Das Stativ 5 kann entweder fest auf dem Boden stehen, wie in Fig. 1 dargestellt oder, wie in Fig. 2 skizziert, Rollen aufweisen, so dass die Magnetfeldeinheit 1 leicht bewegt werden kann.

Eine weitere Möglichkeit wäre es die Magnetfeldeinheit 1 beispielsweise mittels einer Führungsschiene in vertikaler Richtung beweglich an einer Halterung 19 an einer Wand zu befestigen.

Die Beweglichkeit der Magnetfeldeinheit 1 in vertikaler Richtung ermöglicht es, einen Patient P vor dem Stativ 5 sitzend oder stehend zu positionieren, bevor man die Magnetfeldeinheit 1 von oben um den Kopf des Patienten P herum bringt. Ausserdem kann die Lage der Magnetfeldeinheit 1 in vertikaler Richtung an die Größe des Patienten P angepasst werden. Dabei ist die Längsachse A der Magnetfeldeinheit 1 in vertikaler Richtung ausgerichtet, wie auch die Hochachse B des Patienten P, wobei mit Hochachse B des Patienten P die senkrecht zu der Frankfurter Horizontalen C und durch deren Flächenschwerpunkt verlaufende Achse bezeichnet wird.

Zur Positionierung des Patienten P kann ein Stuhl vorgesehen sein. Weiterhin können Griffe 7 vorgesehen sein, an denen sich der Patient P während einer Aufnahme mit den Händen festhält. Diese können beispielsweise mittels einer Führung 8 in vertikaler Richtung verschiebbar an dem Stativ 5 befestigt sein, so dass ihre Lage in vertikaler Richtung an die Größe des Patienten P angepasst werden kann.

Weiterhin kann zur Positionierung ein Aufbiss 9 vorgesehen sein. Dieser kann beispielsweise ebenfalls an der Führung 8 befestigt sein, um seine Lage in vertikaler Richtung an den Patienten P anzupassen. Auch eine Führung 10 in horizontaler Richtung kann vorgesehen sein, so dass der Abstand des Patienten P zum Stativ 5 einstellbar ist und damit auch die Lage der Hochachse B des Patienten P relativ zur Lage der Längsachse A der Magnetfeldeinheit 1.

Weiterhin kann eine Vorrichtung zur Fixierung 20 des Patienten P vorgesehen sein, die beispielsweise eine Stirnstütze 11 und/oder Ohroliven 12 umfasst und die beispielsweise ebenfalls an der Führung 8 angebracht sind. Ein Schema einer entsprechenden Vorrichtung zur Fixierung 20 ist auch in Fig. 3 dargestellt. Es besteht weiterhin die Möglichkeit Teile der Magnetfeldeinheit 1, wie die Vorrichtung 3 zur Erzeugung eines Gradientenfeldes oder die Spule 4 zum Erzeugen und Empfangen von Hochfrequenzen oder auch eine reine Empfangsspule 13 an der Vorrichtung zum Fixieren 20 anzuordnen. (Die vorliegende Erfindung sieht jedoch vor, die Spulen der Vorrichtung zur Erzeugung eines Gradientenfeldes und die mindestens eine Spule zum Erzeugen und Empfangen von Hochfrequenzen in Zwischenräumen zwischen den axial beabstandeten Permanentmagneten anzuordnen.) Wie in Fig. 3 dargestellt, könnte beispielsweise eine als planare Oberflächenspule ausgebildete Empfangsspule 13 so an der Vorrichtung zur Fixierung 20 angeordnet sein, dass sie sich seitlich sehr nah am aufzunehmenden Kiefer K des positionierten Patienten P befinden. Dies kann die Qualität des Messsignals erhöhen.

Um die Sensitivität der Empfangsspule 13 zu erhöhen, kann diese auf dem Aufbiss 9 angeordnet werden, so dass sie sich während einer Aufnahme möglichst nah an dem zu vermessenden Objekt, also dem Kiefer K des Patienten P befindet. Auch das Anordnen der Vorrichtung 3 zur Erzeugung eines Gradientenfeldes an dem Aufbiss 9 kann vorteilhaft sein, da die beispielsweise in Form von Spulen ausgebildete Vorrichtung 3 dadurch ortsfest mit dem zu vermessenden Objekt, also dem Kiefer K verbunden ist, sich also zu vermessendes Objekt und Vorrichtung 3 gleich bewegen, so dass Fehler oder Ungenauigkeiten in der Ortsauflösung der Vermessung, die durch eine relative Bewegung des zu vermessenden Objekts gegenüber dem Gradientenfeld entstehen, vermieden werden.

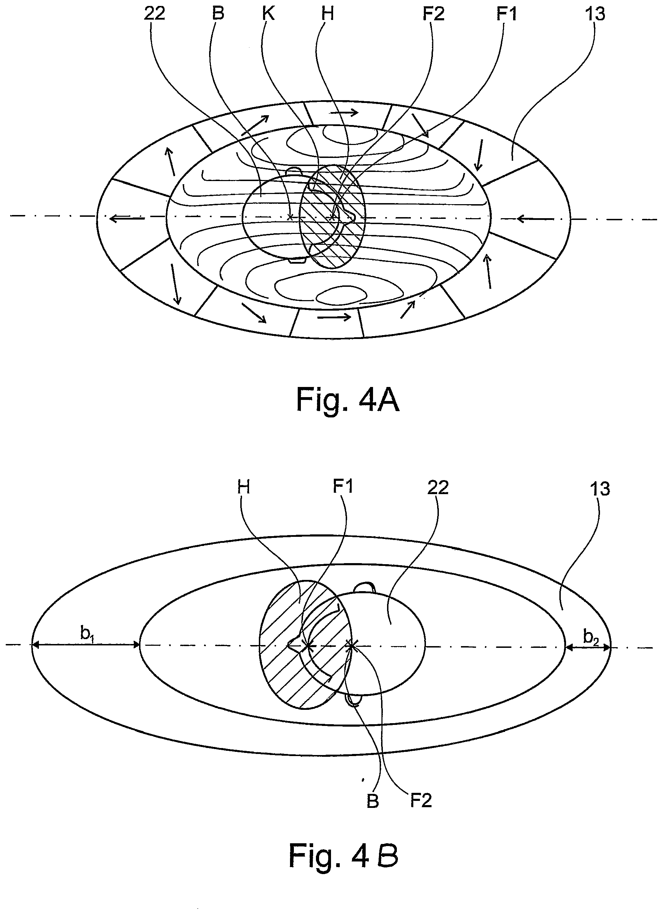

Die Form des Permanentmagneten 2 der in Fig. 1 und 2 dargestellten Magnetfeldeinheit 1 kann beispielsweise eine Halbach-Geometrie sein, wie sie in Fig. 4A dargestellt ist, wobei die Linien im inneren den Verlauf des Magnetfeldes skizzieren.

Durch die längliche Form des Permanentmagneten 2 ist es möglich einen Patienten P nicht mittig zu positionieren, sondern mit einem Abstand zwischen der Hochachse B des Patienten P und einem Flächenschwerpunkt F2 der vom Permanentmagneten 2 eingeschlossenen Fläche. Dadurch ist es möglich eine einfache Geometrie des Permanentmagneten 2 zu wählen , ohne dabei den Permanentmagneten 2 unnötig gross ausbilden zu müssen. Weiterhin wird erreicht, dass sich der zu vermessende Bereich, nämlich der Kiefer K des Patienten P, im Bereich des Flächenschwerpunktes der vom Permanentmagneten 2 eingeschlossenen Fläche und damit auch im homogenen Bereich H des Hauptmagnetfeldes liegt.

Die Außenmaße des Permanentmagneten 2 richten sich vor allem danach, dass eine möglichst kompakte Bauweise angestrebt wird und gleichzeitig sichergestellt werden muss,dass das von dem Permanentmagneten eingeschlossene Volumen ausreichend groß ist, um einen Normkopf aufzunehmen und dass der erzeugte homogene Bereich sich mindestens über den aufzunehmenden Kieferbereich erstreckt.

Eine weitere Ausführungsvariante eines oder mehrerer Permanentmagnete 2 ist in Fig. 4B dargestellt. Hier ist die Form des Permanentmagneten 13 ebenfalls länglich, aber die Dicke b1, b2 des Permanentmagneten 13 an den beiden am weitesten voneinander entfernten Punkten der elliptischen Form ist unterschiedlich (b1>b2). Dadurch bildet sich in der von dem Permanentmagneten 13 eingeschlossenen Fläche ein homogenes Magnetfeld, dessen Flächenschwerpunkt F1 zum Flächenschwerpunkt F2 der eingeschlossenen Fläche beabstandet ist. Dadurch ist es möglich einen Patienten P mittig innerhalb der Permanentmagnete 13 zu positionieren, so dass die Hochachse B des Patienten P durch den Flächenschwerpunkt F2 der vom Permanentmagneten 13 eingeschlossenen Fläche verläuft oder diesem zumindest sehr nahe kommt und gleichzeitig ein örtliche Übereinstimmung des aufzunehmenden Objekts, also des Kiefers K, mit dem homogenen Bereich H des Magnetfeldes der Permanentmagneten 13 zu erreichen.

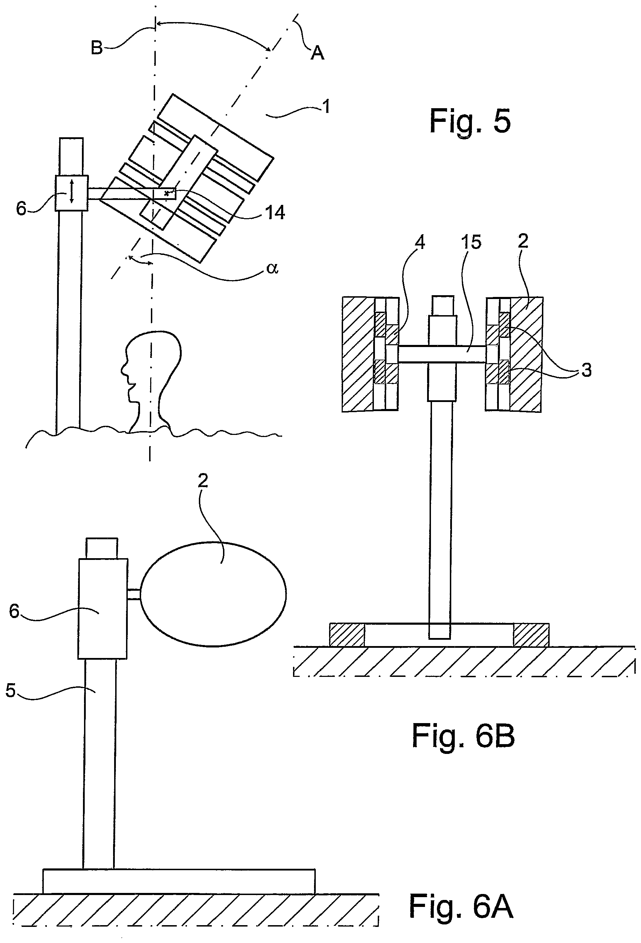

Eine weiteres Ausführungsbeispiel der Magnetfeldeinheit 1 aus Fig. 1 und 2 mit einem geschlossenen Permanentmagneten 2 ist in Fig. 5 dargestellt. Es ist ein Gelenk 14 zwischen Führung 6 und Magnetfeldeinheit 1 mit einer in horizontaler Richtung verlaufenden Gelenkachse vorgesehen, wobei die Gelenkachse senkrecht zur Bildebene verläuft. Dadurch kann die Magnetfeldeinheit 1, also seine Längsachse A, gegenüber der senkrecht verlaufenden Hochachse B eines Patienten P um einen Winkel α von bis zu 45° verkippt werden. Dies kann es ermöglichen, den homogenen Bereich H des Hauptmagnetfeldes des Permanentmagneten 2 besser mit dem zu vermessenden Bereich, also dem Kiefer K des Patienten P, zu überlappen.

Es wäre ebenso möglich die Magnetfeldeinheit 1 fest an dem Stativ 5 oder an der Halterung 19 an der Wand zu befestigen und den Patienten P beispielsweise mittels eines in vertikaler Richtung verfahrbaren Stuhls in die Magnetfeldeinheit 1 hinein zu bewegen.

### Bezugszeichenliste

- 1: Magnetfeldeinheit
- 2: Permanentmagnet
- 3: Vorrichtung zur Erzeugung eines Gradientenfeldes
- 4: Spule zum Erzeugen und Empfangen von Hochfrequenzen
- 5: Stativ
- 6: vertikale Führung
- 7: Griff
- 8: vertikale Führung
- 9: Aufbiss
- 10: horizontale Führung
- 11: Stirnstütze
- 12: Ohroliven
- 13: Empfangsspule
- 14: Gelenk
- 15: Joch
- 16: Ausgleichsspulensystem
- 17: Steuereinheit
- 18: MRT-System
- 19: Halterung
- 20: Vorrichtung zur Fixierung
- 21: Abschirmvorrichtung
- 22: Normkopf
- A: Längsachse der Magnetfeldeinheit 1
- α: Winkel
- B: Hochachse des Patienten P
- C: Frankfurter Horizontale
- F1: Flächenschwerpunkt des homogenen Bereichs H
- F2: Flächenschwerpunkt der vom Permanentmagneten 2 einge schlossenen Fläche
- H: homogener Bereich des Hauptmagnetfeldes
- K: Kiefer des Patienten P
- P: Patient

## Patentansprüche

1. Magnetfeldeinheit (1) eines MRT-Systems (18) zur bildgebenden Erfassung eines Kopfbereichs als aufzunehmender Bereich, umfassend eine Permanentmagnetanordnung (2) zur Erzeugung eines Hauptmagnetfeldes, eine Halterung (19) für die Permanentmagnetanordnung, eine aus mehreren Spulen bestehende Vorrichtung (3) zur Erzeugung eines Gradientenfeldes, sowie mindestens eine Spule (4) zum Erzeugen und Empfangen von Hochfrequenzen, wobei sich innerhalb des von der Permanentmagnetanordnung (2) eingeschlossenen Volumens ein dreidimensionaler homogener Bereich (H) des Hauptmagnetfeldes ausbildet und wobei die Permanentmagnetanordnung so an der Halterung (19) befestigt ist, dass eine Längsachse (A) der Permanentmagnetanordnung (2) mit einer in vertikaler Richtung verlaufenden Achse einen Winkel (α) von maximal ±45° einschliesst, so dass erreicht werden kann, dass die Längsachse (A) der Permanentmagnetanordnung (2) und eine Hochachse (B) eines sitzenden oder stehenden Patienten (P) maximal einen Winkel (α) von 40° einschliessen, wobei die Permanentmagnetanordnung (2) und der Patient (P) relativ zueinander so verstellbar sind, dass die Permanentmagnetanordnung (2) in eine um den Kopf des Patienten (P) herum verlaufende Lage gebracht werden kann,
**dadurch gekennzeichnet, dass**
die Permanentmagnetanordnung(1)sich aus axial beabstandeten Permanentmagneten mit jeweils einer im Querschnitt senkrecht zu der Längsachse (A) geschlossenen Form zusammensetzt, und
dass die Spulen der Vorrichtung (3) zur Erzeugung eines Gradientenfelds und die mindestens eine Spule (4) zum Erzeugen und Empfangen von Hochfrequenzen in Zwischenräumen zwischen den axial beabstandeten Permanentmagneten (2) angeordnet sind.

2. Magnetfeldeinheit (1) nach Anspruch 1, **dadurch gekennzeichnet, dass** ein von der Permanentmagnetanordnung zumindest teilweise begrenztes Volumen einen Durchmesser aufweist, der maximal dem zweifachen eines Durchmessers eines Normkopfes (22) entspricht.

3. Magnetfeldeinheit (1) nach einem der Ansprüche 1 bis 3, **dadurch gekennzeichnet, dass** der homogene Bereich des erzeugten Hauptmagnetfeldes mindestens so groß ist, dass er die unteren 15 cm eines in der Magnetfeldeinheit (1) positionierten Normkopfes (22) abdeckt.

4. Magnetfeldeinheit (1) nach einem der Ansprüche 1 bis 3, **dadurch gekennzeichnet, dass** die Permanentmagnetanordnung länger als breit ist.

5. Magnetfeldeinheit (1) nach einem der Ansprüche 1 bis 4, **dadurch gekennzeichnet, dass** die Permanentmagnetanordnung so um den Patienten (P) herum anordenbar ist, dass die Hochachse (B) des Patienten (P), also die Achse durch den Flächenschwerpunkt der Frankfurter Horizontalen, zu einem Flächenschwerpunkt (F1) einer zur Längsachse (A) senkrechten Schnittfläche des homogenen Bereichs (H) des Hauptmagnetfeldes beabstandet ist.

6. Magnetfeldeinheit (1) nach einem der Ansprüche 1 bis 5, **dadurch gekennzeichnet**, das ein Flächenschwerpunkt (F1) einer zur Längsachse (A) senkrechten Schnittfläche des homogenen Bereichs (H) des Hauptmagnetfeldes mit einem Flächenschwerpunkt (F2) einer zur Längsachse (A) senkrechten Schnittfläche eines von der Permanentmagnetanordnung eingeschlossenen Volumens übereinstimmt.

7. Magnetfeldeinheit (1) nach einem der Ansprüche 1 bis 6, **dadurch gekennzeichnet, dass** die Permanentmagnetanordnung so um den Patienten (P) herum angeordnet werden kann, dass die Hochachse (B) des Patienten (P) zu einem Flächenschwerpunkt (F2) einer zur Längsachse (A) senkrechten Schnittfläche eines von der Permanentmagnetanordnung eingeschlossenen Volumens beabstandet ist.

8. Magnetfeldeinheit (1) nach einem der Ansprüche 1 bis 7, **dadurch gekennzeichnet, dass** ein Flächenschwerpunkt (F1) einer zur Längsachse (A) senkrechten Schnittfläche des homogenen Bereichs (H) des Hauptmagnetfeldes zu einem Flächenschwerpunkt (F2) einer zur Längsachse (A) senkrechten Schnittfläche des vom Permanentmagneten (2) eingeschlossenen Volumens beabstandet ist.

9. Magnetfeldeinheit (1) nach Anspruch 8, **dadurch gekennzeichnet, dass** die Permanentmagnetanordnung so um den Patienten (P) herum angeordnet werden kann, dass die Hochachse (B) des Patienten (P) mit der Lage des Flächenschwerpunktes (F2) der zur Längsachse (A) senkrechten Schnittfläche des von der Permanentmagnetanordnung eingeschlossenen Volumens übereinstimmt oder dieser nahe liegt, so dass der aufzunehmende Bereich, welcher zur Hochachse (B) des Patienten (P) beabstandet ist, mit der Lage des homogenen Bereichs (H) des Hauptmagnetfeldes übereinstimmt.

10. Magnetfeldeinheit (1) nach einem der Ansprüche 1 bis 9, **dadurch gekennzeichnet, dass** die Permanentmagnetanordnung mittels eines Gelenks (14) gegenüber der Halterung (19) um eine horizontale Achse verkippt werden kann.

11. Magnetfeldeinheit (1) nach einem der Ansprüche 1 bis 10, **dadurch gekennzeichnet, dass** die Permanentmagnetanordnung eine Vorrichtung zur Fixierung (20) eines Kopfes eines Patienten (P) und/oder einen Aufbiss(9) aufweist.

12. Magnetfeldeinheit (1) nach einem der Ansprüche 1 bis 11, **dadurch gekennzeichnet, dass** die Magnetfeldeinheit (1) eine bewegliche Abschirmvorrichtung aufweist, die so um die Permanentmagnetanordnung mit ihrer Halterung (19) und den an der Magnetfeldeinheit (1) positionierten Patienten (P) herum bringbar ist, dass möglichst keine elektromagnetische Strahlung von aussen in den Bereich innerhalb der Abschirmvorrichtung eindringen kann.

13. Magnetfeldeinheit (1) nach einem der Ansprüche 1 bis 12, **dadurch gekennzeichnet, dass** die Magnetfeldeinheit (1) ein Ausgleichsspulensystem (26) zum Ausgleichen von Störungen des Hauptmagnetfeldes aufweist.

14. Magnetfeldeinheit (1) nach einem der Ansprüche 1 bis 13, **dadurch gekennzeichnet, dass** sie ein Stativ (5) enthält und die Halterung (19) an dem Stativ (5) in vertikaler Richtung beweglich angebracht ist.

## Claims

1. Magnetic field unit (1) of an MRI system (18) for the image-generating detection of a head area as the region to be recorded, comprising a permanent magnet arrangement (2) for generating a primary magnetic field, a mount (19) for the permanent magnet arrangement, an apparatus (3) consisting of a number of coils for generating a gradient field, and at least one coil (4) for generating and receiving high frequencies, wherein a three-dimensional homogeneous region (H) of the primary magnetic field is formed within the volume enclosed by the permanent magnet arrangement (2), the permanent magnet arrangement is fastened to the mount (19) in such a way that a longitudinal axis (A) of the permanent magnet arrangement (2) forms an angle (α) of no more than ± 45° with an axis extending in vertical direction so that the longitudinal axis (A) of the permanent magnet arrangement (2) and a vertical axis (B) of a seated or standing patient (P) at most form an angle (α) of 40°, and wherein the permanent magnet arrangement (2) and the patient (P) can be adjusted relative to one another such that the permanent magnet arrangement (2) can be brought into a position around the head of the patient (P),
**characterized in that**
the permanent magnet arrangement (1) [sic] consists of axially spaced permanent magnets, each having a form which is closed in cross-section perpendicular to the longitudinal axis (A), and
that the coils of the apparatus (3) for generating a gradient field and the at least one coil (4) for generating and receiving high frequencies are disposed in spaces between the axially spaced permanent magnets (2).

2. Magnetic field unit (1) according to Claim 1, **characterized in that** a volume which is at least in part delimited by the permanent magnet arrangement has a diameter that corresponds to a maximum of twice a diameter of a standard head (22).

3. Magnetic field unit (1) according to any one of Claims 1 to 3 [sic], **characterized in that** the homogeneous region of the generated primary magnetic field is at least large enough to cover the lower 15 cm of a standard head (22) positioned in the magnetic field unit (1).

4. Magnetic field unit (1) according to any one of Claims 1 to 3, **characterized in that** the permanent magnet arrangement is longer than it is wide.

5. Magnetic field unit (1) according to any one of Claims 1 to 4, **characterized in that** the permanent magnet arrangement can be arranged around the patient (P) in such a way that the vertical axis (B) of the patient (P), i.e. the axis through the centroid of the area of the Frankfurt horizontal plane, is spaced apart from a centroid (F1) of a sectional area of the homogeneous region (H) of the primary magnetic field which is perpendicular to the longitudinal axis (A).

6. Magnetic field unit (1) according to any one of Claims 1 to 5, **characterized in that** a centroid (F1) of a sectional area of the homogeneous region (H) of the primary magnetic field which is perpendicular to the longitudinal axis (A) coincides with a centroid (F2) of a sectional area of a volume enclosed by the permanent magnet arrangement which is perpendicular to the longitudinal axis (A).

7. Magnetic field unit (1) according to any one of Claims 1 to 6, **characterized in that** the permanent magnet arrangement can be arranged around the patient (P) in such a way that the vertical axis (B) of the patient (P) is spaced apart from a centroid (F2) of a sectional area of a volume enclosed by the permanent magnet arrangement which is perpendicular to the longitudinal axis (A).

8. Magnetic field unit (1) according to any one of Claims 1 to 7, **characterized in that** a centroid (F1) of a sectional area of the homogeneous region (H) of the primary magnetic field which is perpendicular to the longitudinal axis (A) is spaced apart from a centroid (F2) of a sectional area of a volume enclosed by the permanent magnet arrangement (2) which is perpendicular to the longitudinal axis (A).

9. Magnetic field unit (1) according to Claim 8, **characterized in that** the permanent magnet arrangement can be arranged around the patient (P) in such a way that the vertical axis (B) of the patient (P) coincides with or is close to the position of the centroid (F2) of the sectional area of the volume enclosed by the permanent magnet arrangement which is perpendicular to the longitudinal axis (A), so that the region to be recorded, which is spaced apart from the vertical axis (B) of the patient (P), coincides with the position of the homogeneous region (H) of the primary magnetic field.

10. Magnetic field unit (1) according to any one of Claims 1 to 9, **characterized in that** the permanent magnet arrangement can be tilted about a horizontal axis relative to the mount (19) by means of a joint (14).

11. Magnetic field unit (1) according to any one of Claims 1 to 10, **characterized in that** the permanent magnet arrangement comprises an apparatus for immobilizing (20) a head of a patient (P) and/or a bite block (9).

12. Magnetic field unit (1) according to any one of Claims 1 to 11, **characterized in that** the magnetic field unit (1) comprises a movable shielding apparatus, which can be brought into a position around the permanent magnet arrangement and its mount (19) and the patient (P) positioned at the magnetic field unit (1), so as to ideally allow no electromagnetic radiation to penetrate from the outside into the region within the shielding apparatus.

13. Magnetic field unit (1) according to any one of Claims 1 to 12, **characterized in that** the magnetic field unit (1) comprises a compensation coil system (26) to compensate for disturbances of the primary magnetic field.

14. Magnetic field unit (1) according to any one of Claims 1 to 13, **characterized in that** said unit includes a stand (5) and the mount (19) is disposed on the stand (5) so as to be movable in vertical direction.

## Revendications

1. Unité de champ magnétique (1) d'un système de tomographie par résonance magnétique MRT (18) permettant l'acquisition d'une image d'une région de la tête en tant que région à visualiser, comprenant un ensemble d'aimants permanents (2) pour la production d'un champ magnétique principal, un support (19) pour l'ensemble d'aimants permanents, un dispositif (3) constitué de plusieurs bobines pour la production d'un champ de gradient, ainsi qu'au moins une bobine (4) pour la production et la réception de hautes fréquences,
où une région tridimensionnelle homogène (H) du champ magnétique principal se forme à l'intérieur du volume inclus dans l'ensemble d'aimants permanents (2) et où l'ensemble d'aimants permanents est fixé au support (19) de telle manière qu'un axe longitudinal (A) de l'ensemble d'aimants permanents (2) forme avec l'axe s'étendant dans la direction verticale un angle (α) d'au maximum ± 45° de sorte qu'il peut arriver que l'axe longitudinal (A) de l'ensemble d'aimants permanents (2) et l'axe vertical (B) d'un patient (P) assis ou debout forment au maximum un angle (α) de 40°, l'ensemble d'aimants permanents (2) et le patient (P) pouvant être réglés l'un par rapport à l'autre de telle manière que l'ensemble d'aimants permanents (2) puisse être amené dans une position faisant le tour de la tête du patient (P),
**caractérisée en ce que**
l'ensemble d'aimants permanents (1) se compose d'aimants permanents espacés axialement avec respectivement une forme fermée verticalement dans la section transversale par rapport à l'axe longitudinal (A), et
**que** les bobines du dispositif (3) pour la production d'un champ de gradient et l'au moins une bobine (4) pour la production et la réception de hautes fréquences sont disposées dans des espaces intermédiaires entre les aimants permanents (2) espacés axialement.

2. Unité de champ magnétique (1) selon la revendication 1, **caractérisée en ce qu'**un volume délimité au moins partiellement par l'ensemble d'aimants permanents présente un diamètre qui correspond au maximum à deux fois un diamètre d'une tête normalisée(22).

3. Unité de champ magnétique (1) selon l'une des revendications 1 à 3, **caractérisée en ce que** la région homogène du champ magnétique principal produit est au moins aussi grande qu'elle recouvre les 15 cm les plus bas d'une tête normalisée (22) positionnée dans l'unité de champ magnétique (1).

4. Unité de champ magnétique (1) selon l'une des revendications 1 à 3, **caractérisée en ce que** l'ensemble d'aimants permanents est plus long que large.

5. Unité de champ magnétique (1) selon l'une des revendications 1 à 4, **caractérisée en ce que** l'ensemble d'aimants permanents peut être disposé autour du patient (P) de telle manière que l'axe vertical (B) du patient (P), c'est à dire l'axe à travers le barycentre du plan de Francfort est espacé par rapport à un barycentre (F1) d'une section perpendiculaire à l'axe longitudinal (A) de la région homogène (H) du champ magnétique principal.

6. Unité de champ magnétique (1) selon l'une des revendications 1 à 5, **caractérisée en ce qu'**un barycentre (F1) d'une section perpendiculaire à l'axe longitudinal (A) de la région homogène (H) du champ magnétique principal correspond à un barycentre (F2) d'une section perpendiculaire à l'axe longitudinal (A) d'un volume inclus dans l'ensemble d'aimants permanents.

7. Unité de champ magnétique (1) selon l'une des revendications 1 à 6, **caractérisée en ce que** l'ensemble d'aimants permanents peut être disposé autour du patient (P) de telle manière que l'axe vertical (B) du patient (P) est espacé par rapport à un barycentre (F2) d'une section perpendiculaire à l'axe longitudinal (A) d'un volume inclus dans l'ensemble d'aimants permanents.

8. Unité de champ magnétique (1) selon l'une des revendications 1 à 7, **caractérisée en ce qu'**un barycentre (F1) d'une section perpendiculaire à l'axe longitudinal (A) de la région homogène (H) du champ magnétique principal est espacé par rapport à un barycentre (F2) d'une section perpendiculaire à l'axe longitudinal (A) du volume inclus dans les aimants permanents (2).

9. Unité de champ magnétique (1) selon la revendication 8, **caractérisée en ce que** l'ensemble d'aimants permanents peut être disposé autour du patient (P) de telle manière que l'axe vertical (B) du patient (P) correspond à la position du barycentre (F2) d'une section perpendiculaire à l'axe longitudinal (A) d'un volume inclus dans l'ensemble d'aimants permanents ou est proche de celui-ci de sorte que la région à visualiser, laquelle est espacée par rapport à l'axe vertical (B) du patient (P), correspond à la position de la région homogène (H) du champ magnétique principal.

10. Unité de champ magnétique (1) selon l'une des revendications 1 à 9, **caractérisée en ce que** l'ensemble d'aimants permanents peut être basculé autour d'un axe horizontal au moyen d'une articulation (14) en face du support (19).

11. Unité de champ magnétique (1) selon l'une des revendications 1 à 10, **caractérisée en ce que** l'ensemble d'aimants permanents présente un dispositif pour la fixation (20) d'une tête d'un patient (P) et/ou d'un appareil d'orthodontie (9).

12. Unité de champ magnétique (1) selon l'une des revendications 1 à 11, **caractérisée en ce que** l'ensemble d'aimants permanents (1) présente un dispositif de blindage qui peut être placé autour de l'ensemble d'aimants permanents avec son support (19) et autour du patient (P) positionné dans l'unité de champ magnétique (1) de sorte qu'aucun rayonnement électromagnétique ne peut pénétrer dans la région à l'intérieur du dispositif de blindage à partir de l'extérieur.

13. Unité de champ magnétique (1) selon l'une des revendications 1 à 12, **caractérisée en ce que** l'unité de champ magnétique (1) présente un système de bobines de compensation (26) pour compenser les troubles du champ magnétique principal.

14. Unité de champ magnétique (1) selon l'une des revendications 1 à 13, **caractérisée en ce qu'**elle contient un trépied (5) et **en ce que** le support (19) est rapporté mobile en direction verticale sur le trépied (5) .
